# EUROPEAN PATENT APPLICATION

(11) **EP 2 468 219 A1**
(43) Date of publication of application: **27.06.2012**
(21) Application number: 11177297.6
(22) Date of filing: 11.08.2011
(51) Int. Cl.: A61F 5/01

(54) **Body positioning aid for body alignment**

(30) Priority: 11.08.2010 US 372647 P
(71) Applicant: Al Mubayadh, Ibrahim Abdulqader Abdullah, 31952 Al-Khobar (SA)
(72) Inventor: Al Mubayadh, Ibrahim Abdulqader Abdullah, 31952 Al-Khobar (SA)
(74) Representative: Findlay, Alice Rosemary

(57) **Abstract**

A body positioning aid may include one or more cushions optionally comprising one or more fasteners for securing said one or more cushions to at least one of an inner surface of a leg covering, an outside surface of a leg covering and a leg. The one or more cushions may extend a distance at least sufficient to extend from a user's medial knee and extend to said user's medial malleolus and may be configured to maintain contact with said user's medial knee and user's medial malleolus.

## Description

The invention relates to methods, devices, and apparatuses for preventing and alleviating body pain and promoting body alignment. In particular, the invention provides one or more cushions adapted to be mounted to, or otherwise secured to, or in contact with a subject's body or clothing in order to facilitate proper alignment of the body including but not limited to the spine, hips, pelvis and trunk while also facilitating relaxation of the user's muscles, as well as promoting body alignment, blood circulation, and other benefits while sleeping or resting.

Sleep problems and lower back pain (LBP), and body pain are serious issues. For example, patients suffering from back pain create significant health-care expenses and the economic impact of LBP is also great. Further, sleep problems such as sleep apnea, snoring and aspiration may be the result of LBP, Moreover, women who are pregnant, those suffering from or recovering from injury, the elderly and many others may experience LBP, various sleep problems, body pain, poor circulation, and various other issues.

There are many products and services that purport to solve these problems Among a class of solutions for the above-mentioned issues, are body pillows Existing body pillows, however, have various disadvantages. For example, they are unable to remain functional, i.e., engaged with the body of the user, when the user changes sleep positions, and may not provide support in needed areas. Indeed, such pillows may cause further disturbed sleep by forcing users to be consciously aware of the body pillow or not supporting the proper areas. Furthermore, traditional body pillows are generally heavy and/or bulky, thereby taking up extra space in the bed and thus encroaching upon or interfering with a user's desired sleep position once he/she changes position.

Thus, there is an essential need for devices, methods, and apparatuses which provide a cushion to align the spine, the lower extremities, and or the body during sleep, Further there is a need to achieve an aligned body trunk, cushioning of the knees and medial malleoli and to support important areas of the user's body. Such a body positioning aid is also needed to remain in position with the user throughout the user's sleep or resting period. Such a body positioning aid may enable users to receive various benefits of the body positioning aid,

In an aspect of the invention, a body positioning aid may include one or more cushions with a fastener for securing the one or more cushions to an inside leg covering, an outside leg covering, a leg and the one or more cushions may extend at least a distance from a user's medial knee and extend to a user's medial malleolus and the one or more cushions may be configured to maintain contact with the user's medial knee and medial malleolus. In embodiments, the one or more cushions may be angled to conform to a user's leg when lying in a relaxed position on his or her side, The one or more cushions may be covered in a material that results in a degree of friction when in contact with an opposing surface. Further, the one or more cushions may include shape memory material and memory foam,

In embodiments, the one or more cushions covered in a material may have a first and second side wherein the first and second side include fasteners capable of fastening to one of a user's right leg, left leg, right leg covering and left leg covering. In embodiments, the opposing surface may be a second cushion covered in a material that results in a degree or friction when in contact with an opposing surface or the opposing surface may be one or more leg coverings. In other embodiments, the one or more cushions may be covered in a material that results in a degree of friction and the material may have attached thereto the fasteners for fastening to at least one of the bare leg of a user, an inner surface of a leg covering and an outside surface of a leg covering. In embodiments where the material has such fasteners, the cushion may or may not contain said fasteners. Further, in embodiments, the fasteners may include straps and may be secured with soft hook and loop fasteners, and in embodiments the straps may be one of velvet, satin and other material.

In embodiments, the thickness of the one or more cushions may include a thickness of at least one of 1.5 and 2 inches, In embodiments, the total thickness of the one or more cushions may not exceed 4 inches.

In an aspect of the invention, the body positioning aid may include one or more leg coverings worn by a user capable of receiving a cushion wherein the cushion is received by one or more leg coverings worn by a user and wherein the one or more cushions extends a distance at least sufficient to extend from the user's medial knee and extend to user's medial malleolus and configured to maintain contact with said user's medial knee and medial malleolus. In embodiments, the one or more leg coverings may include one or more receiving pouches located on an inner surface or on any part of the surface of the leg covering and one or more receiving pouches may be capable of receiving the cushion. Further, in embodiments, the one or more receiving pouches may include a fastener for securing a cushion in a desired position. Further, the receiving pouches of the body positioning aid may include internally placed fasteners for securing the cushion in a desired position and the cushion may include complementary fasteners for coupling to said fasteners of the receiving pouches. The internally placed fasteners and the complementary fasteners may include soft hook and loop fasteners.

In other embodiments, the body positioning aid may include one or more leg coverings worn by a user capable of receiving a cushion wherein the cushion is received by one or more leg coverings worn by a user and wherein the one or more cushions extends a distance at least sufficient to extend from the user's medial knee and extend to user's medial malleolus and configured to maintain contact with said user's medial knee and medial malleolus wherein the one or more leg coverings include one or more receiving pouches capable of receiving a cushion and includes a fastener for securing said cushion in a desired position and wherein the cushion is covered in a material that results in a degree of friction when in contact with an opposing surface. In embodiments, said material may include a fastener for coupling to said receiving pouches. Further, the fasteners may include hook and loop fasteners including soft fabric hook and loop fasteners. In embodiments, the material that results in a degree of friction when in contact with an opposing surface may include a zipper or other means for receiving and securing said cushion.

In an aspect of the invention, the body positioning aid may include one or more leg coverings worn by a user capable of receiving a cushion wherein the cushion is received by one or more leg coverings worn by a user and wherein the one or more cushions extends a distance at least sufficient to extend from the user's medial knee and extend to user's medial malleolus and configured to maintain contact with said user's medial knee and medial malleolus wherein the one or more leg coverings include one or more receiving pouches capable of receiving a cushion and includes a fastener for securing said cushion in a desired position and wherein the cushion is covered in a material that results in a degree of friction when in contact with an opposing surface, and in embodiments, the material may include fasteners for removably coupling to said one or more leg coverings. In embodiments, the one ore more cushions may include a first and second side wherein the first and second side include fasteners capable of fastening to a user's right leg covering and a user's left leg covering. Further, the opposing surface may be one or more of said leg coverings, and a second cushion covered in a material that results in a degree of friction when in contact with an opposing surface. In aspects of the intention, the cushion may be angled to conform to a user's leg when lying in a relaxed position on his or her side. Further the cushion may extend the length of a user's leg to include covering the user's medial malleolus. In embodiments, the cushion may include memory material and specifically foam memory material. The cushion may include a thickness of at least one of 1.5 and 2 inches and in embodiments, the cushion and/or cushions may not exceed a total thickness of 4 inches. In embodiments the positioning aid may separate the legs a distance of only 4 inches. Further, in embodiments, the positioning aid may further include at least one or more leg coverings including fasteners for receiving said cushion and said cushion including fasteners for coupling to said one ore more leg coverings.

Methods of the present invention may include methods of aligning the body and other methods including providing one or more cushions and coupling the one or more cushions to at least on of an inner surface of a leg covering and a leg wherein the one or more cushions extends a distance at least sufficient to extend from a user's medial knee and extend to said user's medial malleolus. Further, methods may include one or more cushions covered in a material that results in a degree of friction when in contact with an opposing surface. Methods may include one or more of said leg coverings being capable of receiving the one or more cushions by removably coupling to said cushion via one or more fasteners. Furthermore, methods may include a cushion angled to conform to a user's leg when said user is in a relaxed position on said user's side. Methods may include on or more leg coverings receiving the cushion in a receiving pouch.

These and other systems, methods, objects, features, and advantages of the present invention will be apparent to those skilled in the art from the following detailed description of the preferred embodiment and the drawings,

All documents mentioned herein are hereby incorporated in their entirety by reference. References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context.

The invention will now be further described by way of example with reference to the accompanying drawings, in which:

Figs, 1 depicts cushions of an embodiment of the invention.

Fig. 2 depicts cushions of an embodiment of the invention.

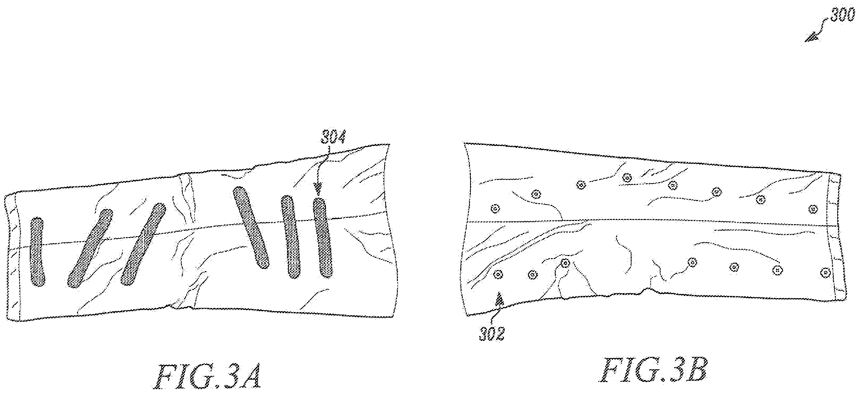

Fig. 3 depicts leg coverings of an embodiment of the invention.

Fig. 4 depicts a leg covering, pants, and receiving pouch of an embodiment of the present invention.

Fig. 5 depicts an embodiment of the present invention.

Fig. 6 depicts an embodiment of the present invention.

Fig. 7 depicts benefits of an embodiment of the present invention.

Fig. 8A and 8b depict the effect of alignment.

Fig. 9 depicts benefits of an embodiment of the present invention.

Fig. 10 depicts a cushion of an embodiment of the invention.

Fig, 11 depicts an embodiment of the present invention.

Embodiments of the present invention will become more fully apparent from the following description and appended claims, taken in conjunction with the accompanying figures. Understanding that the figures merely depict exemplary embodiments of the present invention they are, therefore, not to be considered limiting of its scope. It will be readily appreciated that the components of the embodiments of the present invention, as generally described and illustrated herein, could be arranged and designed in a wide variety of different configurations, Nonetheless, certain embodiments of the invention will be described and explained with additional specificity and detail through the use of the accompanying figures and text herein.

The present disclosure provides a cushion that is intended to be used in a pair or by itself to cushion the space between the legs extending at least from a user's knees to their medial malleoli to provide body positioning, spinal alignment, and impact point cushioning. The cushion may be shaped and sized for comfort, accurate fit, and optimal cushioning. There may be various ways for the cushions to be secured to the user in practice, such as fasteners on the cushion or a covering of the cushion thereof that couple with a fastener on a leg covering or the leg itself, a receiving pouch for the cushion on an inner surface of the leg covering, built-in to the leg covering, or through a frictional fit between the cushion/cushion cover and the leg/leg covering.

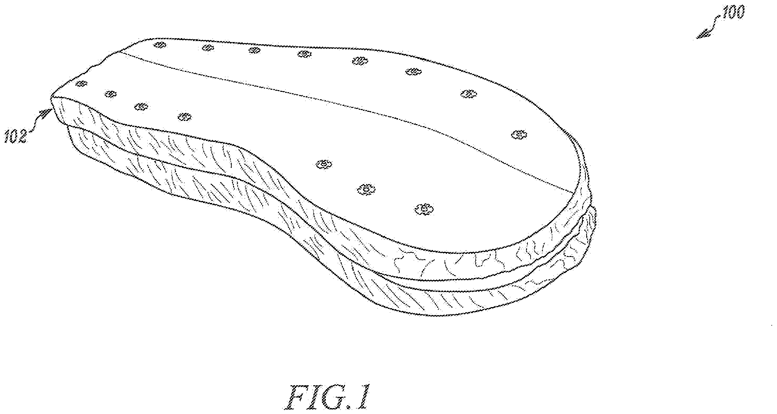

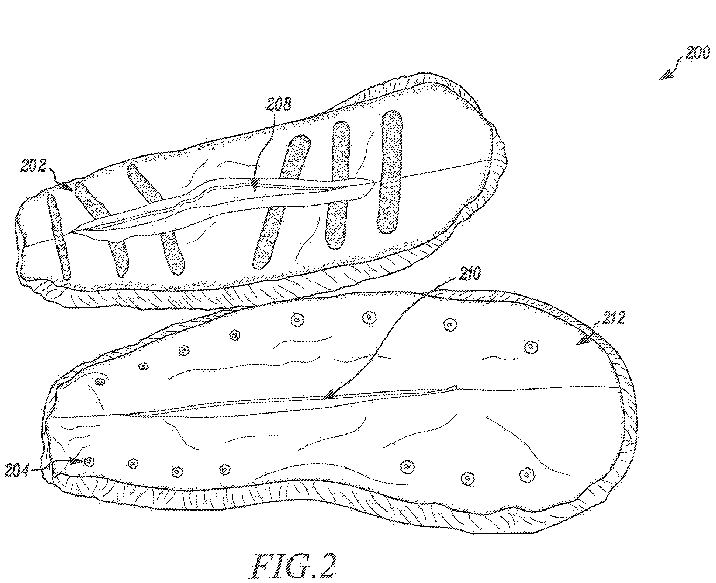

Referring to Fig. 1. the positioning aid may comprise a pair of cushions and coverings. A pair of cushions and cushion coverings 100 is depicted stacked together. The single cushion and cushion covering 102 is shown. Fig. 2 shows two cushions side-by-side 200. A cushion 208 is seen inside of a cushion covering 212. Such cushions may be made of low-density memory foam, memory foam, or other elastomeric material, The cushions may be shaped to align with the leg when the leg is in a relaxed position during side sleeping. Such cushions may be shaped ergonomically to a user's leg. The cushion 208 or cushion and cushion covering 102 may be provided in various lengths for various users and to cover various portions of a user's leg. In embodiments the cushion 208 or cushion and cushion covering 102 may extend a length of at least the user's medial knee to cover the user's medial malleolus, The cushion and cushion cover lengths may be of varying lengths to accommodate various users and user's needs. In embodiments, each cushion and cushion covering 102 and or cushion 208 may be provided in a variety of thicknesses such as 2.0" and 1.5". In embodiments, the thickness of all cushions used may be uniform or they may differ from one another. In various embodiments, other thicknesses of the cushion 208 and or cushion and cushion covering 102 may be used, In certain embodiments, the total thickness of the two cushions and cushion coverings 100 may not exceed 4", Similarly, where cushions 208 are used without cushion coverings 212, such thickness may not exceed 4" in certain embodiments. The cushion covering 212 may be adapted to fit various cushions 208 to accommodate a desired attribute as described herein.

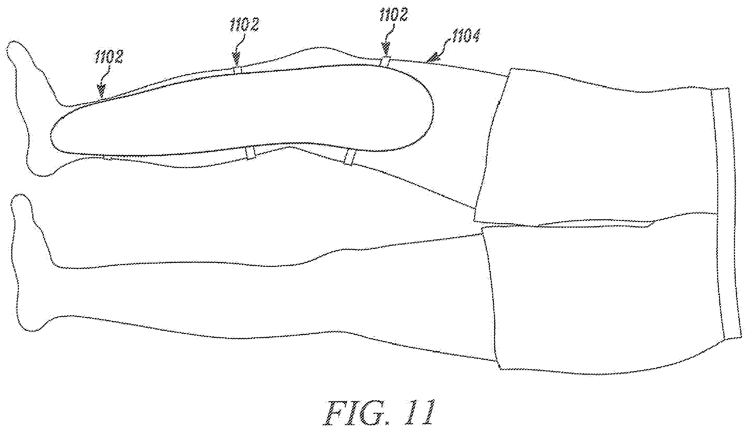

In embodiments, one or more cushions may be provided in a cushion covering 212 made from stretch velvet or other suitable fabric. The cushion covering 212 may result in a degree of friction when in contact with an opposing surface. Referring again to Fig. 2, such cushion covering 212 may contain an opening 210 with a zipper or other means for creating an opening for receiving and securing said cushion and then closing said opening 210. For example, a hook and loop fastener may be used at said opening 210 and create a means for inserting and securing the cushion 208 into the covering 212. In embodiments, the covering 212 may contain hook and loop fasteners, buttons, snaps or other fastening means 202, 204 for securing the cushion and covering 102 to another object such as leg covering described further herein. Further, the cushion covering 212 may be shaped to receive the foam or other elastomeric material ergonomically shaped to a user's leg. Referring to Fig. 11, the cushion and cushion covering 102 may contain straps or bands 1102 for securing the cushion and cushion covering to one or more of a user's leg. In embodiments, the cushion alone may contain straps or bands 1102 for securing the cushion to one or more of a user's leg 1104.

Referring to Figs, 3A and 3B, embodiments of the present invention may include one or more leg coverings 300. The leg coverings 300 may contain fastening means 302, 304 to which the cushion and cushion covering 102 may be fastened. The fastening means may be hook and loop fasteners, snaps, buttons and the like. In embodiments, a cushion and cushion covering 102 may be fastened to each leg covering. The fastening means 302,304 may be complementary to fasteners 202, 204 found on cushion covering 212 or they may be complementary to fasteners found on the cushion 208 itself.

In embodiments, one or more leg coverings 300 may receive one or more cushions 208. In embodiments one or more leg coverings 300 may receive one or more cushions and cushion coverings 102. As used herein, a leg covering and/or a receiving pouch 402 may be described as "receiving" a cushion 208 or cushion and cushion covering 102 to describe that either (102,208) is fastened or attached to the leg covering 300 or inserted into the receiving pouch 402. Further, the cushion 208 or cushion and cushion covering 102 may be described as "coupled" to a leg covering 300 or a user's leg. Further, the leg or leg covering 300 may be described as coupled to the cushion 208 or cushion and cushion covering 102 and the like and this may also be described as fastening or attaching to the other as described herein.

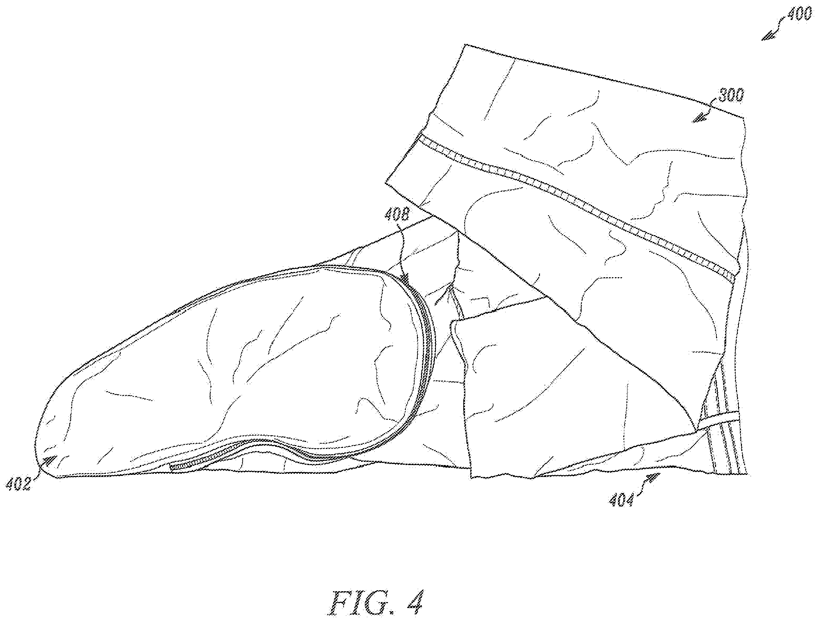

Referring to Fig. 4, the leg covering 300 may be part of a pair of pants 404. In embodiments, pants 404 may be sweat pants, pajama pants, pajama jeans and the like. The pants may be a unisex, for males, or for females and may be various sizes The pants may be designed in various styles and to satisfy locally desired attributes. Leg coverings 300 may contain one or more receiving pouches 402. A pair of pants 404 may comprise one or more leg coverings 300 and one or more receiving pouches 402. Pants 404 and receiving pouch 402 may make up a pants and receiving pouch combination 400. Each leg covering 300 on pants 404 may contain one or more receiving pouches 402 Such receiving pouch 402 may be sewn or otherwise fastened to leg covering 300. The receiving pouch may be sewn or fastened to the inner portion of the leg covering. In embodiments, the receiving pouch may be placed such that it is in between the leg coverings such that it is in between the user's legs. In embodiments, the receiving pouch 402 may be comprised of a material that results in a degree of friction when in contact with an opposing surface. In embodiments, the receiving pouch may contain a zipper or other fastener 408 for securing cushion 208 or cushion and cushion covering 102 inside of the receiving pouch, In embodiments, fasteners may be positioned inside of the receiving pouch 402 for coupling to corresponding fasteners on cushion 208 or cushion and covering 102. In embodiments, receiving pouch 402 may contain hook and loop or other fasteners on the outside surface of the receiving pouch 402 for coupling to corresponding fasteners on the cushion 208 or cushion and covering 102 for securing the cushion 208 in a desired position. In embodiments, the corresponding fasteners may be on the cushion 208 and on the cushion and covering 102. Such fasteners on the cushion and cushion covering 102 are represented by element number 202 and 204 in Fig. 2. In embodiments, fasteners may be hook and loops, buttons, clasps, snaps, zippers and the like. In embodiments, such fasteners may also be made from soft fabric in a hook and loop mechanism or other material suitable for comfort during sleep. In embodiments, the pattern of such fasteners may be standardized. In embodiments, fasteners as described herein may be of any combination and number as suitable for the embodiment. Further in embodiments, the positioning of the fasteners may be such that the a cushion and cushion covering 102 will only correspond to that of the left or right leg covering or left or right receiving pouch. In other embodiments, the cushion and cushion covering 102 may be received by either leg covering or receiving pouch. In embodiments, the receiving pouch 402 may extend the entire length of a user's leg, from at least the users knee to cover the user's medial malleolus. In embodiments, the receiving pouch may be another length and in yet other embodiments, the length of the receiving pouch may be standardized. In embodiments, the size, volume, or height of the receiving pouch may be standardized regardless of the thickness of the memory foam. In other embodiments, the receiving pouch may be fitted to receive a cushion of a particular size.

Accordingly, as a non-limiting example of an embodiment of the invention, the body positioning aid may be comprised of a pair of pajama pants worn by a user where the pants contain a pouch on one or both legs. The receiving pouches may contain a cushion or cushion and covering or a combination thereof. Such receiving pouch may be positioned on the inside of the user's leg or legs such that the cushion or cushion and covering may be received and positioned between a user's legs. The cushions may provide cushioning from at least the user's knee down to the user's ankle to cover the medial malleolus. The cushion may be shaped such that it contours or follows the user's leg when in a side sleeping position. The cushion may be ergonomically shaped to the user's leg or legs in that the shape may align the trunk and or lover extremities of the body and cushion the knees and medial malleoli in any posture while side sleeping. The cushion, cushion and covering, and receiving pouch may extend below the bottom of the pants by at least some distance. In embodiments, the distance may be two inches, another distance or the distance appropriate for a user to cover the user's medial malleolus. Further, the positioning aid may contain cushions fastened to the inside of each of a user's leg such that the cushions are opposed to one another and between the user's legs when in a side sleeping position.

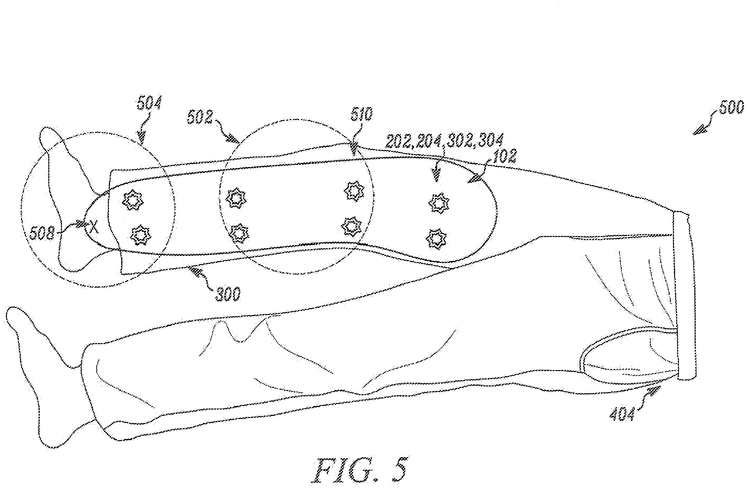

Referring to Fig. 5, one or more cushion and cushion covering 102 may be secured directly to one or more leg coverings 300 on pants 404 as shown together as positioning aid 500. In embodiments, the cushion 208 may be secured to the leg covering 300. The cushion 208 and or cushion and cushion covering 102 may be secured via any fastening means such as snaps, a hook and loop mechanism, buttons and the like, Such fasteners may be found on the cushion covering represented by element number 202 and 204 and corresponding fasteners may be found on the leg coverings as represented by element number 302, 304. Cushion cover fasteners 202, 204 and leg covering fasteners 302,304 may be complementary and may be comprised of material that will not disturb the user during sleep. In embodiments, cushion fasteners (not shown) and leg covering fasteners 302,304 may be complementary and may be comprised of material that will not disturb the user during sleep. In embodiments described herein, such material may be soft hook and loop fasteners, plush buttons, soft plastic snaps and the like. Further, any other suitable fastener may be used.

Again referring to Fig. 5, the cushion and cushion covering 102, or cushion alone 208 may be fastened to leg covering 300 on pants 404 to form a cushion and pants positioning aid 500. In embodiments, the cushion and cushion covering 102 may extend from the user's knee or medial knee, or at a point above the user's knee or medial knee past the bottom of the pants to cover the user's medial malleolus 508. Such covering may cushion impact points represented by circles and element numbers 502 and 504, and may provide support to the user's knee at contact point 510 and user's medial malleolus at 508. In embodiments, said cushion and cushion covering 102 may extend beyond the leg covering 300 by two inches or a desired length appropriate to provide support to the user. In various embodiments the cushion 208, cushion and covering 102, with or with out a receiving pouch 402 may extend from any point above, or beginning at the user's medial knee down along the user's leg to cushion the medial malleolus 508. In embodiments, such cushioning may be provided with pants 404 or with a user's bare leg 1104 as seen in Fig. 11. Further, such cushioning may be provided with the cushion and cushion covering 102 fastened to the leg covering 300 with fasteners 202,204 and 302,304 and/ or received in a receiving pouch 402, In embodiments, a combination of fastening means may be used. The cushion and cushion covering 102 may be received in a receiving pouch 402 on one leg and on the other, the cushion and cushion covering 102 may be fastened to the leg covering 300 via fasteners 202,204 and 302,304. The cushion and cushion covering 102 may be received by two leg coverings 300 via fasteners as described above. Conversely, the cushion and cushion covering 102 may be received by a receiving pouch 402 on both leg coverings 300, The cushion 208 or cushion and cushion covering 102 may be shaped such that it extends the entire the length of the user's leg to the user's foot or any other length. The positioning aid may employ one cushion to be placed on a user's leg and between the user's legs or the positioning aid may be comprised of two cushions, one on each leg of the user fastened by various means. In embodiments described herein, a cushion 208 without a cushion covering 212 may be used in place of the cushion and cushion covering 102.

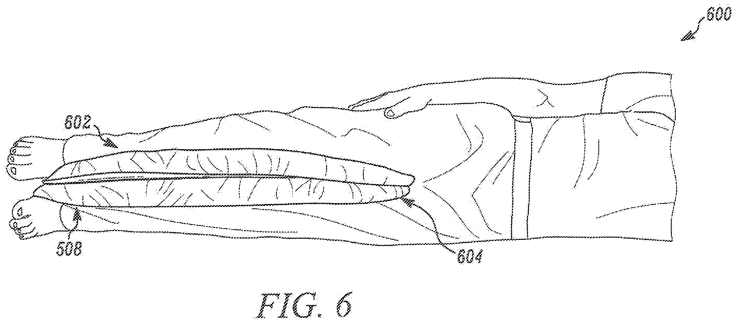

Referring to Fig. 6, a user in a side sleeping position 600 may use a positioning aid comprising two cushions and cushion coverings 100. Such a positioning aid 602 may extend from the user's upper leg region 604 down to the user's medial malleolus 508. In embodiments, the positioning aid 602 may extend only to cover the user's ankle such that the user may walk comfortably while wearing the positioning aid 602. In embodiments, the positioning aid may be various lengths such that it covers the entire leg or various lengths of the leg, The positioning aid may also be made to accommodate various lengths to fit children and adults of various sizes. Each cushion of the pair of cushions and coverings 100 may be the same length as the other in the pair or the lengths may differ from the other. In embodiments, the cushions of the positioning aid may be any desired thickness to accommodate various user preferences and various weights of user's extremities. Further, in embodiments, the pair of cushions and coverings 100 of positioning aid 602 may be received by leg coverings 300 via a receiving pouch 402, various fasteners, straps and the like. In embodiments, the pair of cushions and coverings 100 of positioning aid may be received by a user's bare leg. In various embodiments described herein, one or more cushions 208 without a cushion covering 212 may be used in place of one or more cushion and cushion coverings 102.

Referring to Fig. 11, one or more cushions 208 or cushions and cushion covering 102 may be fastened to a user's bare leg in the event the user wishes to use the positioning aid without pants 404 as described in Fig. 4. The cushions may be fastened to a user's bare leg 1104 via straps or bands 1102. Such bands may be fastened with hook and loops, buttons, snaps, clips and the like. In embodiments, the straps or bands 1102 may be elastic bands capable of securing to the user's leg without additional fastening means, In embodiments, the straps or bands 1102 may be made from satin, velvet or other material. In embodiments, the user may fasten a single cushion and cushion covering 102 to each bare leg to create a positioning aid similar to that of Fig. 6 differing only in the lack of use of leg covering, In various embodiments described herein, one or more cushions 208 without a cushion covering 212 may be used in place of one or more cushion and cushion coverings 102.

In the various embodiments described here, friction, including friction between one or more cushions, the cushion and the leg covering, the cushion and the user's leg and the like, may provide extra stability and support which may enhance the apparatus's ability to decrease or eliminate lower extremities misalignment which may consequently result in aligned spine and relaxed back muscle system, In various embodiments, the cushion covering 212 may be made from any material that may cause friction such as velvet normally used for memory foam pillows or other suitable fabric that creates friction. Other materials may also be used to obtain friction including polymers, materials having a polymer coating, latex, or textured materials. Such frictional material may be provided or otherwise applied to the cushions via a bag. sleeve, or other container. Such friction may help keep the positioning aid in place throughout a user's entire night of sleep and while turning and moving during sleep. This may decrease a user's awareness of the positioning aid by allowing the user to use the aid without having to be concerned with the positioning aid being shifted from its desired position.

in embodiments described above and those apparent to one skilled in the art, the positioning aid may be attached to a user in a manner consistent with providing more comfortable sleep. By way of example, such positioning aid may be fastened to the user in such as way as to provide a means of keeping it in place during a full period of sleep even while the user moves about his or her various sleeping position, in this way, the user may not be subconsciously, consciously or otherwise aware of the positioning aid and may not be concerned with keeping it in place throughout his or her sleep period which may result in a more restful period of sleep, For example after putting the positioning aid in place, the user may not need to physically reposition the positioning aid during his or her sleep.

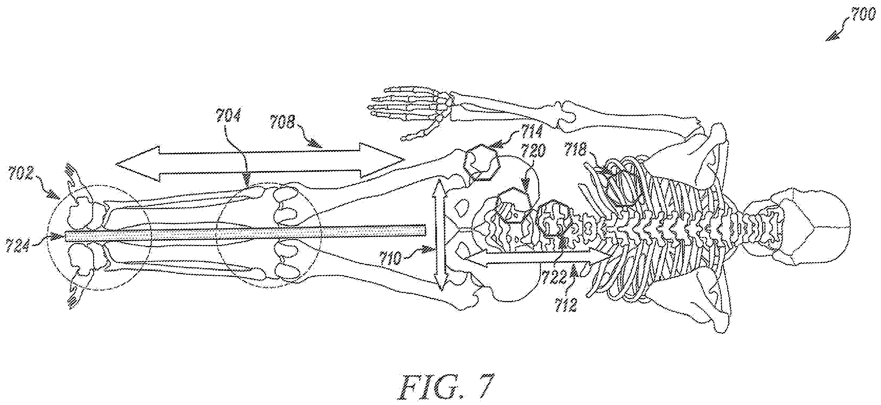

Referring to Fig. 7, the positioning aid may provide support to the user and aid in muscular, skeletal and overall body alignment Referring to the skeleton 700 and when used in a position such as that represented by, but not limited to position 724, the user may experience lower extremity alignment 708, general body and lower back alignment 712 and hips and pelvis alignment 710. Use of such positioning aid as described herein may reduce the: impact on the hips 714, impact on the back muscle system 718, impact on the sacroiliac joints 720, and the impact on the lumber vertebrae and facet joints 722. Further, use of positioning aids as described herein may reduce the impact at the knees 704 and ankles and medial malleoli 702 of the user.

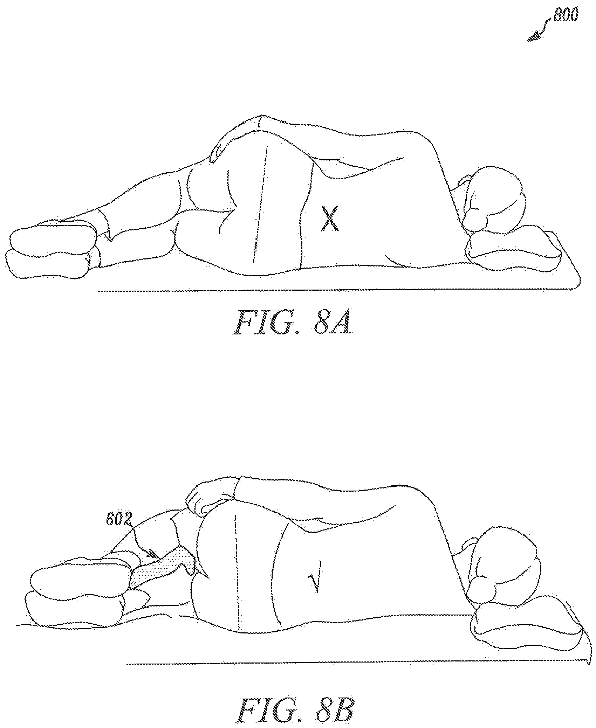

Referring to Fig. 8A and 8B, a companion of a user's body alignment is shown with and without use of the positioning aid 602. As shown in Fig. 8A, the side sleeper 800 may not experience general body alignment when in a side sleeping position Further, the user may not experience lower back alignment and hip and pelvis alignment. Conversely, as depicted in Fig. 8B, the side sleeper 800 may experience body alignment when using a positioning aid as described herein. Although positioning aid 602 is depicted, one skilled in the art would understand that any embodiment of the positioning aids described herein may be suitable for use in Fig, 8b.

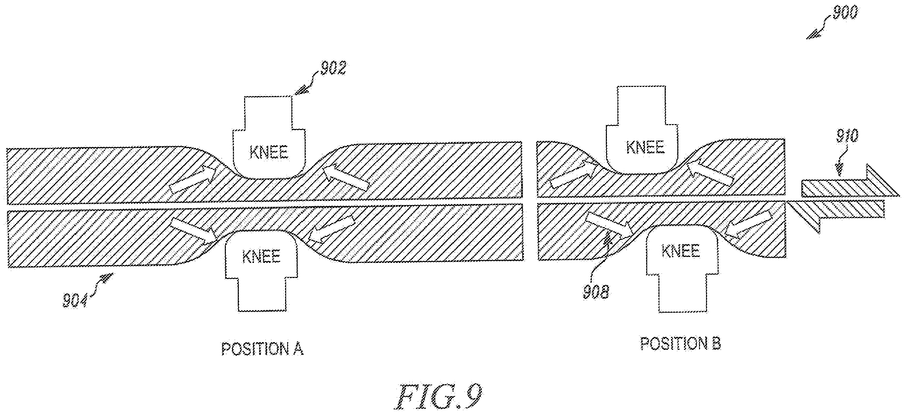

Fig, 9 depicts an embodiment 900 of the present invention. Specifically, Fig. 9 depicts a cushion 904 that may or may not be in a cushion covering 212. The user's body may take desired position A or position B as described in Fig. 9. In such cases, the foam may create pressure 908 that allows for stability and alignment of user's body, Cushion 904 may stabilize users knees 902 and medial malleoli (not shown), In embodiments, the stabilization of the medial malleoli and/or knees promotes the desired alignment and muscle relaxation benefits of the present invention. In embodiments, the stabilization of the medial malleoli and/or knees combined with the cushioning of the medial knee and medial malleoli promotes the desired alignment and muscle relaxation benefits of the present invention. The cushion may shape ergonomically to the user's body to support and align the body. In embodiments, the cushion 904 may create a separation between a user's medial malleoli and or knees or medial knees of a desired dimension. Further, said cushion may be comprised of foam or may be covered as described previously herein with a velvet, fabric or other covering that provides friction 910 to limit sliding of the cushion. Such friction 910 may limit sliding of the cushion and allow for maximum effectiveness and alignment. Although positioning aid 900 is depicted, one skilled in the art would understand that any embodiment of the positioning aids described herein may be suitable for such use and to provide the benefits described.

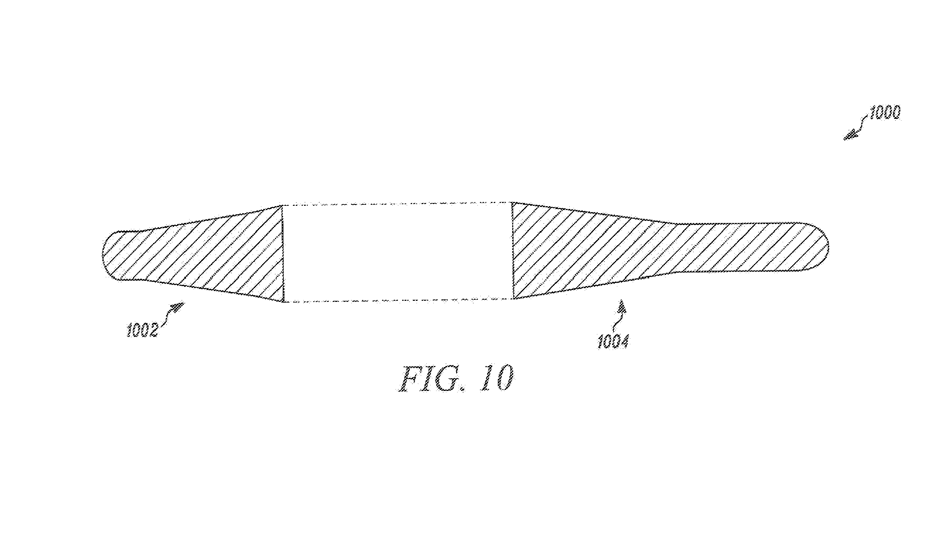

Referring to Fig. 10, cushion 1000 depicts a cross section of a cushion of an embodiment of the present invention. In embodiments, upper end 1004 may be three to four inches long and the foot end 1002 may be two inches long. However, in various embodiments, such upper end 1004 and foot end 1002 may be various lengths. Additionally, the length of the cushion 1000 may be various lengths and various thicknesses at different points along the length of the cushion. In embodiments, the foot end 1002 and the upper end 1004 may be tapered or un-tapered. In embodiments, the thickness of the cushion may be reduced at both ends to the thickness accepted by industry standards. In embodiments, the cushion 1000 may be shaped to accommodate fastening to a leg.

Use of the positioning aid may allow the cushions to accommodate or provide cushioning to pressure points on the legs, such as the knees, medial knees, and medial malleoli, which in turn provides lower extremity alignment, body-lower back alignment, and hips and pelvis alignment. With respect to the medial malleoli, the cushions may extend a distance sufficient to accommodate the medial malleoli, such distance being beyond the end of the pajama bottom or other leg covering in embodiments. The cushions may be provided in a variety of sizes to accommodate different users.

In embodiments, the cushions may be secured to the inside surface of clothing, e.g. pajama bottoms such that the cushions contact the inside surface of the leg or to an outside surface of the pajama or leg covering. Alternatively, as discussed herein, the cushions may be directly secured to the leg of a user such that the pair of cushions oppose each other, while each contact the inside surface of the leg to which the cushion is attached. The cushions as described herein, cushion coverings, leg coverings, and pants as may be provided with mating zipper sections. Velcro® sections, snaps, hook and loop fasteners, buttons or combinations thereof in order to secure the cushions to the user and or the leg coverings/pants. Alternatively, each cushion may also be provided with a securing device as described below in connection with one-cushion embodiments. In embodiments, one cushion may be fastened to a leg covering while the other cushion is fastened directly to the leg. Any combination of fastening to the inside surface of the leg covering, the outside surface of the leg covering, and directly to the leg for the pair of cushions is contemplated by the invention,

In embodiments, utilization of low-density memory foam, or memory foam enables the positioning aid to achieve mechanics necessary for alignment of the back and lower extremities. In embodiments, the cushions may be configured to space apart, at a predetermined distance, the section of a user's leg extending from the user's knees or medial knees to the user's medial malleoli. In embodiments, the distance chosen to space apart the portions of the user's legs may be optimized for providing alignment to various parts of the user's body or for alignment of the user's entire body, In embodiments, the distance achieved by the cushion between the user's knees may be 4 inches. In embodiments, the distance achieved by the cushion between the user's medial malleoli may be 4 inches. The memory foam acts also as an inter-leg cushion. Friction, including friction between the cushion, the pajama and the cushions themselves provides extra stability and support as well as to eliminate lower extremities misalignment which and consequently results in aligned spine and relaxed back muscle system. The cushions may be provided with a material that provides friction, such as a velvet normally used for memory foam pillows or other suitable fabric. Other materials may also be used to obtain friction including polymers, materials having a polymer coating, latex, or textured materials. Such frictional material may be provided or otherwise applied to the cushions via a bag, sleeve, or other container. In embodiments using bags, containers, or sleeves, such bags, containers, or sleeves may be of a standard size that can accommodate varying sizes of the cushions.

Methods of aligning the body and or alleviating ortreating body pain may comprise providing the elements of the positioning aid as described herein. Methods may also comprise pairing or coupling the elements as described herein for a particular user and or to achieve various embodiments as described herein.

Various embodiments of the positioning aid as described herein may be beneficial to various people. For example those suffering from lower back pain and back problems, elderly, prenatal mothers, those suffering from snoring or sleep apnea, those who experience palpitation/aspiration during back sleep, those who need to sleep in a side position, cachectic patients, those recovering from various injuries, and others may benefit from the use of the embodiments of the positioning aid described herein. For example, the embodiments described herein may align the lower back, pelvis and lower extremities and prevent back twisting during sleep to improve sleep quality. The positioning aid may address trunk and lower extremity alignment, align the knees hips and spine, stabilize the body, relax the back and muscles, create customized support to the hip and increase ability to cope with head alignment and align the body. The positioning aid may address trunk and lower extremity alignment by providing an appropriate thickness of the cushions of the body positioning aid as described herein. Embodiments herein provide support during sleep when muscle tone is minimal and when body support is needed. The embodiments described herein may help users recover from injuries and align the spine to treat lower back problems, Those with lower back pain, herniated disks, chronic sacroiliac and lumbar facet joint inflammation and osteoporosis may use the positioning aid to get relief from such ailments and or to recover from such ailments. Embodiments described herein may be used to treat injuries such as spine, hip, lower limb fractures and the like where immobilization is required. Further, such embodiments may be useful in providing support to post operative patients, those who are pregnant and those suffering from lower back pain, and those suffering from pelvis and spine trauma. Embodiments of the positioning aid described herein may maximize recovery, prevent further problems, promote good blood circulation, provide pain and spasm relief, relieve muscle tension, and provide comfortable sleep. The embodiments described herein may contribute to lower back pain disease recovery, back problem prevention and quality sleep.

Various embodiments of the present invention may provide cushioning and support to a user. In particular, a user may experience cushioning at the medial malleolus, the knee and medial knee. Further, a user may experience alignment from the medial malleoli to the medial knee. The positioning aid may provide natural alignment of the pelvis and spine when in resting or sleeping and inhibit spinal twisting during sleep. Embodiments described herein may also provide a decreased impact on the knees and ankles as cushioning is provided between the knees and ankles as described in various embodiments of the invention where one or more cushions comprise the positioning aid. Further, friction as described above provides increased stability for alignment and periods of uninterrupted alignment, which may be attributed to the stabilized positioning of the cushions due to the increased friction, Further, the fastening means and some exemplary embodiments as described herein may eliminate the need to have front knee padding incorporated into the device. In embodiments, the positioning aid may massage and or provide support and comfort to the popliteal of the user. The positioning aid may conform with contours of the popliteal and support and or comfort the area when user bends his or her leg, has his or her leg straight, alternates between positions or otherwise. In embodiments, the width of the cushion may allow the cushion to conform to support and or comfort the user's popliteal as the user bends his leg in a sleeping position or otherwise, straightens his leg, alternates between positions and the like. In embodiments, the cushion may comprise additional material for such support and or may contain an alternate shape for such support. In embodiments, such positioning aid may also help alleviate arthritis pain for side sleepers.

The embodiments described herein may allow the positioning aid to stay in position with the user as he or she changes sleep position, This may result in better sleep for the user as he may be less aware of the positioning aid and as he is able to be less concerned with keeping the positioning aid in place. Further, embodiments of the positioning aid as described herein may be less cumbersome than those currently in the field while providing support and alignment as described herein.

Further, embodiments herein may provide a lighter weight device and a smaller size as compared to other devices in the field. The embodiments as described herein may provide for a compact positioning aid that may be easily packed for travel.

The inventor has found that the invention works very well in any leg position when side sleeping. The results are notable in terms of helping to relieve pain and/or to improve sleep quality.

While the invention has been disclosed in connection with the preferred embodiments shown and described in detail, various modifications and improvements thereon will become readily apparent to those skilled in the art Accordingly, the spirit and scope of the present invention is not to be limited by the foregoing examples, but is to be understood in the broadest sense allowable by law.

## Claims

1. A body positioning aid comprising:
one or more cushions comprising one or more fasteners for securing said one or more cushions to at least one of an inner surface of a leg covering, an outside surface of a leg covering, and a leg, and
wherein said one or more cushions extends a distance at least sufficient to extend from a user's medial knee and extend to said user's medial malleolus and configured to maintain contact with said user's medial knee and user's medial malleolus.

2. The body positioning aid of claim 1 wherein the said one or more cushions is angled to conform to a user's leg when said user is lying in a relaxed position on said user's side.

3. The body positioning aid of claim 1 wherein the one or more cushions is covered in a material that results in a degree of friction when in contact with an opposing surface.

4. The body positioning aid of claim 1 wherein the one or more cushions is comprised of shape memory material

5. The body positioning aid of claim 1 wherein the shape memory material is foam.

6. The body positioning aid of claim 1 wherein the one ore more cushions comprise a first side and second side wherein the first and second side comprise fasteners capable of fastening to at least one of a user's right leg, left leg, right leg covering, and left leg covering.

7. The body positioning aid of claim 3 wherein the opposing surface is a second cushion covered In a material that results in a degree of friction when in contact with an opposing surface,

8. The body positioning aid of claim 3 wherein the opposing surface is one or more of said leg coverings.

9. The body positioning aid of claim 3 wherein the material has attached thereto
said
one or more fasteners for fastening to at least one of a bare leg of said user, an inner surface of a leg covering, and an outside surface of a leg covering.

10. The body positioning aid of claim 9 wherein said fasteners comprise straps, wherein said straps are secured with soft hook and loop fasteners, and wherein said straps are comprised of one of velvet, satin and other material.

11. The body positioning aid of claim 1 wherein the one or more cushions comprise a thickness of at least one of 1 .5 and 2 inches.

12. The body positioning aid of claim 1 wherein the total thickness of the one or more cushions does not exceed 4 inches.

13. A body positioning aid comprising:
one or more leg coverings able to be worn by a user, each of said one or more leg coverings capable of receiving a cushion,
wherein said cushion is received by one or more leg coverings able to be worn by said user, and
wherein said cushion extends a distance at least sufficient to extend from a user's medial knee and extend to said user's medial malleolus and configured to maintain contact with said user's medial knee and user's medial malleolus,

14. The body positioning aid of claim 13 wherein the one or more leg coverings comprises,
one or more receiving pouches located on an inner surface of said leg covering, and
wherein one or more receiving pouches is capable of receiving said cushion.

15. The body positioning aid of claim 14 wherein the one or more receiving pouches comprises a fastener for securing said cushion in a desired position.

16. The body positioning aid of claim 15 wherein the one or more receiving pouches comprises internally placed fasteners for securing said cushion in a desired position, and
wherein said cushion comprises complementary fasteners for coupling to said fasteners on said receiving pouches.

17. The body positioning aid of claim 16 wherein said internally placed fasteners and complementary fasteners comprise soft hook and loop fasteners.

18. The body positioning aid of claim 15 wherein said cushion is covered in a material that results in a degree of friction when in contact with an opposing surface, and
wherein said material that results in a degree of friction when in contact with an opposing surface comprises a fastener for coupling to said receiving pouches.

19. The body positioning aid of claim 15 wherein said fasteners are comprised of hook and loop fasteners comprised of soft fabric,

20. The body positioning aid of claim 18 wherein said material that results in a degree of friction when in contact with an opposing surface further comprises a zipper or other means for receiving and securing said cushion.

21. The body positioning aid of claim 13 comprising:
said cushion covered in a material that results in a degree of friction when in contact with an opposing surface,
the one or more leg coverings comprising fasteners for receiving said cushion covered in said material that results in a degree of friction when in contact with an opposing surface, and
said material that results in a degree of friction when in contact with an opposing surface comprising fasteners for removably coupling to said one or more leg coverings.

22. The body positioning aid of claim 21 wherein said cushion comprises a first side and second side wherein the first and second side comprise fasteners capable of fastening to a user's right leg covering and a user's left leg covering.

23. The body positioning aid of claim 21 wherein the opposing surface is one or more of said leg coverings,

24. The body positioning aid of claim 21 wherein the opposing surface is a second cushion covered in a material that results in a degree of friction when in contact with an opposing surface,

25. The body positioning aid of claim 13 wherein the cushion is angled to conform to a user's leg when said user is lying in a relaxed position on said user's side.

26. The body positioning aid of claim 13 wherein the cushion extends the length of the user's leg to include covering the user's medial malleolus.

27. The body positioning aid of claim 13 wherein the cushion is comprised of memory material.

28. The body positioning aid of claim 13 wherein the memory material is foam.

29. The body positioning aid of claim 13 wherein the cushion received comprises a thickness of at least one of 1.5 and 2 inches.

30. The body positioning aid of claim 13 wherein the positioning aid separates the legs a distance of no more than 4 inches.

31. The body positioning aid of claim 13 further comprising at least one of said one or more leg coverings comprising fasteners for receiving said cushion and said cushion comprising fasteners for coupling to said one or more leg coverings.

32. A method of aligning the body comprising:
providing one or more cushions, and
coupling the one or more cushions to at least one of an inner surface of a leg covering, an outside surface of a leg covering, and a leg,
wherein said one or more cushions extends a distance at least sufficient to extend from a user's medial knee and extend to said user's medial malleolus and configured to maintain contact with said user's medial knee and user's medial malleolus,

33. The method of claim 32 wherein one or more cushions is covered in a material that results in a degree of friction when in contact with an opposing surface.

34. The method of claim 32 wherein one or more of said leg coverings is capable of receiving the one or more cushions by removably coupling to said cushion via one or more fasteners.

35. The method of claim 32 wherein the cushion is angled to conform to a user's leg when said user is lying in a relaxed position on said user's side.

36. The method of claim 32 wherein the one of more leg coverings receives the one or more cushions in a receiving pouch.
